# EUROPEAN PATENT APPLICATION

(11) **EP 3 808 777 A1**
(43) Date of publication of application: **21.04.2021**
(21) Application number: 19203680.4
(22) Date of filing: 16.10.2019
(51) Int. Cl.: C07K 16/42, A61K 9/08, A61K 39/395

(54) **STABLE LIQUID ANTIBODY FORMULATIONS**

(71) Applicant: Glenmark Specialty S.A., 2300 La Chaux-de-Fonds (CH)
(72) Inventor: Dubey, Sachin, 2300 La Chaux-de-Fonds (CH)

(57) **Abstract**

The present invention relates to stable formulations of a therapeutic protein and in particular an antibody, comprising the therapeutic protein, a histidine buffer and at least one stabilizing agent.

## Description

The present invention relates to stable formulations of a therapeutic protein and in particular an antibody, comprising the therapeutic protein, a buffer and/or a stabilizing agent.

### Background of the Invention

The formulation of antibody is a critical aspect of an antibody's use in the treatment of a disease as it allows the formulated antibody to be shipped and stored prior to use as well as allowing its administration to patients. Antibodies as with all therapeutic proteins are prone to degradation, when in a liquid formulation they are also liable to aggregation and/or undesired chemical modifications. The stability of an antibody in liquid formulation depends not only on the kind of excipients used in the formulation, but also on the amount and proportion of the excipients relative to one another. Furthermore, other considerations apart from stability must be taken into account while preparing a liquid antibody formulation. Examples of such additional considerations includes viscosity of the solution, which is dependent in part upon the concentration of antibody that can be accommodated by the other components of the formulation. Thus, while formulating a therapeutic antibody, great care must be taken to arrive at a formulation that remains stable, contains an adequate concentration of antibody, and possesses a suitable viscosity as well as other properties which enable the formulation to be conveniently administered to patients.

In addition a suitable formulation for one therapeutic antibody is not necessarily suitable for a second antibody, due to the physiochemical properties of each antibody that are dependent upon its amino acid composition as well as post-translational modifications, which vary from antibody to antibody.

The high protein concentrations pose challenges relating to the physical and chemical stability of the protein, as well as difficulty with manufacture, storage, and delivery of the protein formulation. The other problem is the tendency of proteins to form particulates during processing and/or storage, which make manipulation during further processing difficult. To attempt to obviate this problem, surfactants and/or sugars have been added to protein formulations. Although surfactants and sugars may reduce the degree of particulate formation of proteins, they do not address another problem associated with manipulating and administering the concentrated protein formulation, i.e., increased viscosity. In fact, sugars may enhance the intermolecular interactions within a protein or between proteins and increase the viscosity of the protein formulation.

Currently omalizumab is present in two different formulations i.e. lyophilized and liquid formulation. The antibody concentration is 150mg/ml and method of administration is subcutaneous. WO97/04801 describes a stable lyophilized formulation of anti-IgE antibodies. WO02/30463 describes reduced-viscosity formulation containing specific salts and a reconstituted anti-IgE mAb, but with a maximum antibody concentration of only up to about 140 mg/mL with addition of salt like NaCl. WO02/096457describes E25 anti-IgE mAb formulation containing acetate/acetic acid buffer with antibody concentration up to 257 mg/mL. WO 2004/091658 describe the Anti IgE liquid formulation which contains the antibody concentration upto 150 mg/ml without salt. WO2011/090088 describe the use of an acidic amino acid i.e. aspartic acid or glutamic acid as a counter ion species in histidine buffer to achieve the stable highly-concentrated antibody-containing formulations and require additonal pH adjustment.

The present invention describe the stable high concentrated anti IgE formulation which contain histidine and high concentration of acidic and basic amino acid which does not require pH adjustment.

### Summary of the Invention

The present invention discloses stable liquid formulations comprising an anti IgE antibody such as omalizumab or a biosimilar thereof. The disclosed formulations were developed in accordance with a defined set of selection criteria based on data collected from analytical procedures performed to evaluate the biochemical and biophysical stability of anti IgE antibody in alternative formulations. Various pH adjustment solutions were screened.

More specifically, the present invention provides stable liquid formulations for an anti IgE antibody referred to herein as omalizumab or its biosimilars.

In one embodiment, the invention provides stable liquid pharmaceutical formulations comprising a histidine buffer. For example, the invention provides stable liquid aqueous pharmaceutical formulation comprising an anti-IgE antibody, a pH-adjustment solution, and a surfactant, wherein the anti- IgE antibody is an omalizumab. The disclosed histidine-buffered formulations can comprise at least 50mg, preferably at least 75mg or from 100 to 200 mg/mL of an anti-IgE antibody. The pH of the disclosed pharmaceutical formulations is between pH 5 to 6., 4 or preferably around pH 5.5.

In an alternative embodiment the invention provide a stable liquid aqueous formulation comprising omalizumab at a concentration of at least 50mg, preferably at least 75mg or from 100 to 200 mg/mL, about 10-30 mM histidine buffer, 200-300 mM glutamic acid, 200-300mM lysine and 0.02%polysorbate 20, wherein the pH of the formulation is about 6.

In an alternative embodiment the invention provide a stable liquid aqueous formulation comprising omalizumab at a concentration of at least 50mg, preferably at least 75mg or from 100 to 200 mg/mL, about 10-30mM histidine buffer, 200-300mM glutamic acid, 200-300mM arginine and 0.02% polysorbate 20, wherein the pH of the formulation is about 6.

In an alternative embodiment the invention provide stable liquid aqueous formulation comprising omalizumab at a concentration of at least 50mg, preferably at least 75mg or from 100 to 200 mg/mL, a 10-30 mM histidine , 400-550 mM arginine and 0.02% polysorbate 20, wherein the pH of the formulation is about 6.

Another aspect of the invention provides a pre-filled syringe or autoinjector device, comprising any of the subject formulations described herein.

In certain embodiments, the aqueous formulation stored in the pre-filled syringe or autoinjector device contains omalizumab at a concentration of at least 50mg, preferably at least 75mg or from 100 to 200 mg/mL in an aqueous formulation

In yet other embodiments, the formulation of omalizumab is defined in terms of the monomer content of the omalizumab antibody under certain conditions. In one embodiment, at 5°C, the % monomer of the omalizumab is > 95%> at 0 month to 18 months as measured by size exclusion chromatography.

In yet other embodiments, the formulation of omalizumab, is defined in terms of the variant content of the omalizumab under certain condition. In further embodiments, a stable liquid aqueous formulation comprising omalizumab at a concentration of at least 50mg, preferably at least 75mg or from 100 to 200 mg/mL, about 10-30mM histidine buffer, 200-300mM glutamic acid, 200-300mM arginine and 0.02% polysorbate 20, wherein the pH of the formulation is about 6, and the % basic variant of the omalizumab is > 10% at 0 to 18 months weeks as measured by ion exchange chromatography.

In further embodiments, stable liquid aqueous formulation comprising omalizumab at a concentration of at least 50mg, preferably at least 75mg or from 100 to 200 mg/mL, about 10-30 mM histidine buffer,200-300 mM glutamic acid, 200-300mMlysine and 0.02%polysorbate 20, wherein the pH of the formulation is about 6 and the % basic variant of the omalizumab is > 10% at 3 to 18 months weeks as measured by ion exchange chromatography.

In a further embodiment, stable liquid aqueous formulation comprising omalizumab at a concentration of at least 50mg, preferably at least 75mg or from 100 to 200 mg/mL, a 10-30 mM histidine , 400-550 mM arginine and 0.02% polysorbate 20, wherein the pH of the formulation is about 6 and the % basic variant of the omalizumab is > 10% at 3 to 18 months weeks as measured by ion exchange chromatography.

In another embodiment, a stable liquid aqueous formulation comprising omalizumab at a concentration of at least 50mg, preferably at least 75mg or from 100 to 200 mg/mL, about 10-30mM histidine buffer, 200-300mM glutamic acid, 200-300mM arginine and 0.02% polysorbate 20, wherein the pH of the formulation is about 6, and the % acidic variant of the omalizumab is less than 15 % at 3 to 18 months weeks as measured by ion exchange chromatography.

In another embodiment, stable liquid aqueous formulation comprising omalizumab at a concentration of at least 50mg, preferably at least 75mg or from 100 to 200 mg/mL, about 10-30 mM histidine buffer,200-300 mM glutamic acid, 200-300mMlysine and 0.02%polysorbate 20, wherein the pH of the formulation is about 6 and the % acidic variant of the omalizumab is less than 15 % at 3 to 18 months weeks as measured by ion exchange chromatography

In a further embodiment, stable liquid aqueous formulation comprising omalizumab at a concentration of at least 50mg, preferably at least 75mg or from 100 to 200 mg/mL, a 10-30 mM histidine, 400-550 mM arginine and 0.02%polysorbate 20, wherein the pH of the formulation is about 6 and the % acidic variant of the omalizumab is less than 15 % at 3 to 18 months weeks as measured by ion exchange chromatography
In a preferred embodiment the formulation comprises 150mg of an antibody such as omalizumab and:
20 mM L-Histidine, 250 mM Glutamic acid, 250 mM Lysine, 0.02% Tween-20 at pH 6.0 with a viscosity of 11.81 cS;
20 mM L-Histidine, 250 mM Glutamic acid, 250 mM Arginine, 0.02% Tween-20 at pH 6.0;
20 mM L-Histidine, 500 mM L-Arginine, 0.02% Tween-20 at pH 6.0 with a viscosity of 6.72 cS.

In these formulations L-hisitidine functions as a buffer, having an effective buffering range of ∼5.5-7.4.

The basic amino acids Lysine and Arginine act as stabilising agents and have no buffering capacity at pH 6, the pKa values being 9.06 and 2.16 for Lysine and 8.99, 1.82 and 12.48 for Arginine.

The acidic amino acid Glutamic Acid acts as a stabilising agent and also has no buffering capacity at pH 6, the pKa values being 9.47 and 2.10.

The surfactant Tween-20 acts to prevent protein denaturation and aggregation.

### Examples

In the following Examples, the present invent ion will be described in detail, the scope of the present Invention is not limited to these Examples.

Samples (Omalizumab formulations) in which various buffer solutions were incorporated were prepared as per table 1

Each of these sample contained 150 Mg/ml of Omalizumab, Histidine buffer, tween-20 and the corresponding one of the pH adjustment solutions shown in table 1 which has pH of 6.

The drug substance of purified omalizumab is obtained and formulated by using various excipient in the present example which is shown in table 1

| Ingredient | Formulation A | Formulation B | Formulation C | Formulation D(Innovator) |
|---|---|---|---|---|
| Omalizumab | 150 | 150 | 150 | 150 |
| L-Histidine | 20 mM | 20 mM | 20mM | 18.4mM |
| Lysine | 250mM | - | - | - |
| Glutamic acid | 250 mM | 250mM | - | - |
| Arginine | - | 250mM | 500mM | |
| Arginine-HCL | - | - | - | 200mM |
| Tween-20 | 0.02% | 0.02% | - | 0.038% |

### Stability studies

All formulations were filled at 1 ml syringe of D Hypak SCF™ PRTC glass pre-fillable syringe. Vials are placed at 5±3°C.

### Analytical method:-

Stability samples were analyzed by the methods outlined below

### Charge Variant Purity by IEC- HPLC-

The IEC is a technique by which biological molecules are separated from each other according to characteristics of charges exposed on the protein Surface. In the present invention, the content (% Acidic) of the acidic product (deamidation product) formed by deamidation of the active main body of omalizumab in each sample was measured and evaluated by using cation-exchange chromatography.

Chromatography was conducted using a Waters 2695 Liquid Chromatography system and a Dionex ProPac® WCX-10 2×250 mm column.

### Size Exclusion Chromatography-

The SEC is a technique by which high-molecular weight species (including aggregates), monomer (active form), and low-molecular weight species (including fragments and degradation products) in a sample are separated from each other by pores of a column packing material. For the evaluation, the monomer content (% Monomer) in each sample was measured and evaluated by using this method.

Chromatography was conducted using a Waters 2695 Liquid Chromatography system and an TOSOH Bioscience TSK gel G3000 SWXLcolumn. The data was analyzed based on relative % areas of main, aggregated and fragmented peaks.

A liquid pharmaceutical formulation having a temperature of 5 ° C ± 2 ° C, a relative humidity of 75 ± 5% and a main component of 98 to 100% as measured by SE-HPLC after storage for 0 - 18 month under confined conditions. A liquid pharmaceutical formulation having a low molecular weight component as measured by SE-HPLC after storage for 0-18 months at a temperature of 5 ° C ± 3 ° C of 0 to 2%;

### Protein Concentrations-

Protein concentration measured by UV- visible spectroscopy. Commonly, the optical absorbance of protein is measured at 280 nm. At this wavelength, the absorbance of protein is mainly due to the amino acids tryptophan, tyrosine and cysteine with their molar absorption coefficients decreasing in that order. Of course, the molar absorption coefficient of the protein itself at 280 nm will depend upon the relative concentrations of these three amino acids. Therefore, different proteins can have different absorption coefficients and even the wavelength of the maximum absorbance may differ. This fact can be used to help identify different types of proteins by relatively fast and simple optical tests.

### Intact immunoglobulin G content (Intact IgG%)

The content (%) of intact immunoglobulin G was measured using Non-Reduced Capillary Electrophoresis-Sodium Dodecyl Sulfate (NR CE-SDS). The principle of the CE-SDS is similar to classical SDS-PAGE technique. However, in CE, the gel is replaced by uncoated capillary (bare fused silica capillary). This analysis separates the light chain, heavy chain and non-glycosylated species by Capillary Gel Electrophoresis in reduced and non- reduced conditions. Both non-reduced and reduced samples are separated through the bare fused capillary and analyzed by PDA detector. The reduced profile is looked for the LC and HC and NG content and the Non- reduced samples are looked for the LC and NG and Whole IgG content To identify the intact Immunoglobulin G content Beckman Coulter PA800 plus system has been used.

### Sub visible particle count

Analysis of sub visible particles are required for final drug product release testing to mitigate the risk associated with the presence of extraneous particles in injectable solutions, particularly the risk of blood vessel occlusion for intravenously administered solutions of small molecule parenteral drug products.

This test is based on Light obscuration method which is the compendia! method (Ph. Eur. 2.9.19 and USP <788>) for the analysis of sub visible particles in parenteral products. In this method, the sample is drawn into the system through a needle and particles pass a laser beam block, a certain amount of light, and produce a "shadow" on a light-sensitive detector. The area of this shadow is then converted to the equivalent circular diameter of the particle and results in to no. of particle counts.

The sub visible particle counter HRLD400 was used for the test.

### Potency analysis

The competition ELISA was used for potency testing. Competitive ELISA involves addition of one more substance to compete with antibody-antigen to bind to the already added antigen-antibody during the reaction. The omalizumab monoclonal antibody inhibits the binding of IgE to the high-affinity IgE receptor FceRl by binding to an epitope on IgE that overlaps with the site to which FceRI binds. This assay is done to detect the binding of Omalizumab monoclonal antibody to its target IgE using a competitive enzyme linked immunosorbent assay (ELISA). The principle is based on the antibody - antigen interaction wherein the antibody or the antigen is adsorbed to a solid surface and will participate in specific high affinity binding. The different working dilutions of the test sample and the reference standard are tested on the plate coated with rhFce-Rla. The detection antibody used is Streptavidin-HRP solution. TMB peroxidase EIA substrate kit used for development. The absorbance is read at 450nm and the EC50 of test sample is compared against the EC50 ofreference standard to evaluate the binding ability of the test sample to its target.

Stability data of formulation A, B and C. All formulation contain 150mg/ml omalizumab.
Formulation A: 20 mM L-Histidine, 250 mM Glutamic acid, 250 mM Lysine, 0.02% Tween-20; pH 6.0

| Real time stability at 5°C | | | | | | |
|---|---|---|---|---|---|---|
| Sr. No | Parameter | Specification | Time point | | | |
| | | | 0 | 6 | 12 | 18 |
| 1 | pH | 6.20 ± 0.3 | 5.96 | 5.91 | 5.91 | 5.97 |
| 2 | Purity by CEX-HPLC | Acidic variant ≤15% | 13.1 | 14.1 | 14.6 | 14.7 |
| | | Main form ≤70% | 69.5 | 68.1 | 65.2 | 66.3 |
| | | Basic form ≤20% | 17.4 | 17.9 | 20.2 | 19.1 |
| 3 | Size Exclusion SEC- | %Monomer Content ≤98% | 98.6 | 98.5 | 98.5 | 98.1 |
| 4 | Protein Concentration mg/ml by UV- Visible Spectroscopy | | 139 | 128 | 135 | 139 |
| 5 | Intact immunoglobulin G content (Intact IgG%) | Reduced - sum heavy and light chain -% | 99% | 99.8% | 99.5 | Not calculated |
| | | Non reduced Main peak% | 94.6% | 96.9% | 95.8% | 95.1% |
| 6 | Subvisible particle (SVP | SVP≥10µm | 1079 | 591 | 173 | 123 |
| | | SVP≥25µm | 31 | 39 | 5 | 4 |
| 7 | Potency | Binding ELISA/KI ELISA | 89 | 79 | 102 | 89 |

Formulation B 20 mM L-Histidine, 250 mM Glutamic acid, 250 mM Arginine, 0.02% Tween-20; pH 6.0

| Real time stability at 5°C | | | | | | |
|---|---|---|---|---|---|---|
| Sr. No | Parameter | Specification | Time point | | | |
| | | | 0 | 6 | 12 | 18 |
| 1 | pH | 6.20 ± 0.3 | 6.09 | 6.17 | 6.21 | 6.34 |
| 2 | Purity by CEX-HPLC | Acidic variant ≤15% | 13.1 | 13.9 | 14.6 | 15.2 |
| | | Main form ≤70% | 68.0 | 71.1 | 69.5 | 70.5 |
| | | Basic form ≤15% | 19 | 15.1 | 16.0 | 14.4 |
| 3 | Size Exclusion SEC- | %Monomer Content ≤98% | 98.2 | 98 | 98 | 97.9 |
| 4 | Protein Concentration mg/ml by UV- Visible Spectroscopy | | 170 | 161 | 153 | 159 |
| 5 | Intact immunoglobulin G content (Intact IgG%) | Reduced - sum heavy and light chain -% | 99.1% | 99.7% | 99.5 | Not calculated |
| | | Non reduced Main peak% | 94.1% | 97.3% | 96.5% | 95.7% |
| 6 | Sub visible particle (SVP | SVP≥10µm | 930 | 619 | 115 | 1158 |
| | | SVP≥25µm | 23 | 28 | 6 | 53 |
| 7 | Potency | Binding ELISA/KI ELISA | 119 | 81 | 82 | 110 |

Formulation C: 20 mM L-Histidine, 500 mM L-Arginine, 0.02% Tween-20; pH 6.0

| Real time stability at 5°C | | | | | | |
|---|---|---|---|---|---|---|
| Sr. No | Parameter | Specification | Time point | | | |
| | | | 0 | 6 | 12 | 18 |
| 1 | pH | 6.20 ± 0.3 | 6.05 | 6.0 | Not recorded | 6.06 |
| 2 | Purity by CEX-HPLC | Acidic variant ≤15% | 12.5 | 13.7 | 14.2 | 14.8 |
| | | Main form ≤70% | 71.6 | 71.5 | 69 | 69.4 |
| | | Basic form ≤15% | 16.0 | 14.9 | 16.8 | 16.0 |
| 3 | Size Exclusion SEC- | %Monomer Content ≤98% | 97.9 | 97.9 | 98.1 | 97.7 |
| 4 | Protein Concentration mg/ml by UV- Visible Spectroscopy | | 158 | 151 | 152 | 153 |
| 5 | Intact immunoglobulin G content (Intact IgG%) | Reduced - sum heavy and light chain -% | 99.1% | 99.7 | Not calculated | Not calculated |
| | | Non reduced Main peak% | 94.1% | 96.4% | | 96.6% |
| 6 | Subvisible particle (SVP | SVP≥10µm | Not calculated | 1313 | Not calculated | 1269 |
| | | SVP≥25µm | Not calculated | 106 | Not calculated | 68 |
| 6 | Potency | Binding ELISA/KI ELISA | 95 | Not calculated | Not calculated | 122 |

Formulation D: 18.4 mM L-Histidine, 200 mM L-Arginine, 0.038% Tween-20; pH 6.0

| Real time stability at 5°C | | | | | | |
|---|---|---|---|---|---|---|
| Sr. No | Parameter | Specification | Time point | | | |
| | | | 0 | 6 | 12 | 18 |
| 1 | pH | 6.20 ± 0.3 | 6.11 | 6.19 | 6.25 | 6.37 |
| 2 | Purity by CEX- HPLC | Acidic variant ≤15% | 13.0 | 13.8 | 14.4 | 15.1 |
| | | Main form ≤70% | 71.6 | 71.8 | 70.5 | 71.7 |
| | | Basic form ≤15% | 15.4 | 14.5 | 15.2 | 13.3 |
| 3 | Size Exclusion SEC- | %Monomer Content ≤98% | 97.9 | 98.0 | 98.1 | 97.8 |
| 4 | Protein Concentration mg/ml by UV-Visible Spectroscopy | | 174 | 151 | 158 | 161 |
| 5 | Subvisible particle (SVP | SVP≥10µm | 803 | 5088 | 172 | 673 |
| | | SVP≥25µm | 14 | 428 | 3 | 34 |
| 6 | Potency | Binding ELISA/KI ELISA | 90 | 79 | 72 | 106 |

## Claims

**1.** A stable liquid aqueous pharmaceutical formulation comprising an anti-IgE antibody, histidine, a pH adjustment solution and a surfactant wherein the pH of the formulation is between about 5.8 to 6.4.

**2.** The formulation of claim 1, wherein the anti IgE antibody present at a concentration of between about 100 and about 200 mg/mL and more specifically between 100 and 170 mg/mL.

**3.** The formulation of claim 1, wherein the pH adjustment solution is selected from Arginine-glutamate, lysine-glutamate or arginine sulfate.

**4.** The formulation of claim 1 wherein the surfactant is polysorbate 20.

**5.** The formulation of claim 1 wherein the anti IgE antibody is omalizumab(Seq id no ??)

**6.** A stable liquid aqueous formulation comprising omalizumab at a concentration of between about 100 and about 170 mg/mL, about 10-30 mM histidine buffer,200-300 mM glutamic acid, 200-300mMlysine and 0.02%polysorbate 20, wherein the pH of the formulation is about 6

**7.** A stable liquid aqueous formulation comprising omalizumab at a concentration of between about 100 and about 170 mg/mL, about 10-30mM histidine buffer, 200-300mM glutamic acid, 200-300mM arginine and 0.02% polysorbate 20, wherein the pH of the formulation is about 6.

**8.** A stable liquid aqueous formulation comprising omalizumab at a concentration of between about 100 and about 170 mg/mL, a 10-30 mMhistidine , 400-550mMarginine and 0.02%polysorbate 20, wherein the pH of the formulation is about 6.

**9.** A pre-filled syringe or autoinjector device, comprising omalizumab at a concentration of between about 100 and 160 mg/ml in an aqueous formulation according to claim 1.

**10.** A pre-filled syringe or autoinjector device, comprising omalizumab at a concentration of between about 100 and 160 mg/ml in an aqueous formulation according to claim 6.

**11.** A pre-filled syringe or autoinjector device, comprising omalizumab at a concentration of between about 100 and 160 mg/ml in an aqueous formulation according to claim 7.

**12.** A pre-filled syringe or autoinjector device, comprising omalizumab at a concentration of between about 100 and 160 mg/ml in an aqueous formulation according to claim 8.

**13.** The formulation of claim 6-8 comprising 20 mM histidine.

**14.** The formulation of claim 6 -7 comprising 250 mM glutamic acid.

**15.** The formulation of claim 6 comprising 250 mM lysine.

**16.** The formulation of claim 7 comprising 250 mM arginine.

**17.** The formulation of claim 8 comprising 500 mM arginine.

**20.** The formulation of claim 6-8 comprising 150mg/ml omalizumab.

**21.** The formulation of any one of claims 1-8, wherein at 40 °C, the % acidic variant of the omalizumab antibody is less than 60% at 18 month as measured by ion exchange chromatography.

**22.** The formulation of any one of claims 1-7, wherein at 40 °C, the % acidic variant of the omalizumab antibody is less than 80% at 18 month as measured by ion exchange chromatography.
